# FASCICULE DE BREVET EUROPEEN

(11) **EP 2 999 406 B1**
(45) Date de publication et mention de la délivrance du brevet: **29.11.2017**
(21) Numéro de dépôt: 14729426.8
(22) Date de dépôt: 14.05.2014
(51) Int. Cl.: A61B 5/091, A61M 16/00, G01F 13/00, G01F 1/115

(54) **DISPOSITIF ET PROCÉDÉ DE MESURE DE DURÉE DE CONSOMMATION DE GAZ DANS UN BÂTIMENT HOSPITALIER**
VORRICHTUNG UND VERFAHREN ZUR MESSUNG EINES GASVERBRAUCHSZEITRAUMS IN EINEM KRANKENHAUSGEBÄUDE
DEVICE AND METHOD FOR MEASURING A GAS CONSUMPTION PERIOD IN A HOSPITAL BUILDING

(30) Priorité: 23.05.2013 FR 1354628
(43) Date de publication de la demande: 30.03.2016
(73) Titulaire: Air Liquide Santé France, 75007 Paris (FR)
(72) Inventeur: BOUCHER, Guillaume, F-94130 Nogent-sur-marne (FR); PAULES, Jérôme, F-75015 Paris (FR)
(74) Mandataire: Pittis, Olivier
(86) Numéro de dépôt international: PCT/FR2014/051116
(87) Numéro de publication internationale: WO 2014/188108

(56) Documents cités:
- EP-A2- 1 983 251
- WO-A2-02/081001
- FR-A1- 2 506 932
- US-B1- 7 890 342

## Description

L'invention concerne un dispositif et un procédé de mesure de la durée de consommation gazeuse adaptés à une utilisation au sein d'un bâtiment hospitalier comprenant un réseau de canalisations de gaz comprenant des prises murales de distribution de gaz agencées sur les murs des chambres des patients, typiquement des prises d'oxygène et d'air médical murales.

Le document WO 02/081001 décrit un dispositif selon l'art antérieur permettant l'enregistrement de la consommation d'un gaz médical par un patient.

Le document EP 1 983 251 décrit un système selon l'art antérieur de gestion de gaz médical prévu pour les installations médicales. Dans les hôpitaux, il est nécessaire de pouvoir suivre la durée de consommations de gaz par les patients, en particulier d'air et d'oxygène, de manière précise et y compris dans les chambres des patients, c'est-à-dire en tête de lit où les gaz sont distribués par des prises d'alimentation murales.

En effet, il est primordial de pouvoir s'assurer que les patients inhalent le gaz qui leur a été prescrit pendant la durée de prescription, c'est-à-dire sans interruption avant la fin de cette durée mais sans aller au-delà de cette durée

Actuellement, lorsqu'un patient inhale du gaz respiratoire, tel de l'oxygène dans le cadre d'un traitement d'oxygénothérapie, délivré par une prise murale alimentée par une canalisation d'amenée de gaz, la détermination de durée d'inhalation du gaz est réalisée de manière empirique par le personnel soignant ou le patient lui-même au moyen d'une montre ou analogue.

Une fois la durée écoulée, le patient retire son masque et/ou le personnel soignant stoppe l'alimentation en gaz du masque respiratoire.

On comprend aisément que cette manière de procéder n'est pas pratique et peut conduire à des erreurs de chronométrage, donc à une administration inadaptée du gaz thérapeutique, et/ou à des pertes de gaz, en particulier lorsque le patient retire son masque mais que l'alimentation dudit masque n'est pas interrompue.

Il est donc nécessaire de pouvoir non seulement déterminer la durée pendant laquelle le patient a inhalé le gaz mais aussi interrompre l'alimentation en gaz du patient lorsque cette durée écoulée correspond à celle de la prescription médicale faite par le médecin.

De là, le problème qui se pose est de proposer un dispositif permettant d'assurer un suivi du temps de consommation précis et une interruption automatique de la distribution du gaz lorsque le patient a reçu suffisamment de gaz pour que sa prescription soit respectée, en particulier lorsque le gaz administré, tel de l'oxygène ou de l'air, est délivré par une prise murale située dans la chambre dudit patient au sein d'un bâtiment hospitalier.

La solution est alors un dispositif de détermination, c'est-à-dire de mesure et de suivi, de la durée de consommation d'un gaz, tel de l'oxygène médical ou de l'air médical, par un patient comprenant :
- un passage interne de gaz avec une (ou plusieurs) entrée de gaz et une (ou plusieurs) sortie de gaz, le gaz circulant dans ledit passage de l'entrée vers la sortie de gaz,
- des moyens de connexion permettant de connecter fluidiquement le dispositif à une prise de distribution de gaz,
- des moyens de réglage permettant de régler une durée de consommation de gaz souhaitée, appelée T_{fixé},
- des moyens d'affichage permettant d'afficher au moins la durée de consommation de gaz souhaitée T_{fixé},
- des moyens de détermination de durée de consommation de gaz conçus pour mesurer la durée totale, appelée T_{conso}, de consommation de gaz pendant laquelle du gaz circule dans ledit passage de gaz, de l'entrée de gaz vers la sortie de gaz,
- des moyens de traitement d'information aptes à et conçus pour comparer la durée T_{conso} totale de consommation de gaz déterminée par les moyens de détermination de durée de consommation de gaz à la durée de consommation de gaz souhaitée T_{fixé}, et
- des moyens d'interruption de flux conçus pour interrompre toute circulation de gaz dans ledit passage de gaz lorsque les moyens de traitement d'information déterminent que la durée T_{conso} totale de consommation de gaz est supérieure ou égale à la durée de consommation de gaz souhaitée T_{fixé}, c'est-à-dire lorsque T_{conso} ≥ T_{fixé}.

Selon le cas, le dispositif de l'invention peut comprendre l'une ou plusieurs des caractéristiques techniques suivantes :
- les moyens de détermination de durée de consommation de gaz comprennent :
   - un organe rotatif apte à et conçu pour être mis en rotation par le flux gazeux circulant dans le passage de gaz, et
   - un capteur à effet hall coopérant avec l'organe rotatif pour comptabiliser la durée pendant laquelle l'organe rotatif est mis en rotation par le flux gazeux circulant dans ledit passage de gaz.
- les moyens de réglage comprennent un ou plusieurs organes de réglage, en particulier une ou des touches, boutons, curseurs, capteurs de pressions ou de présence.
- les moyens d'affichage comprennent un écran ou des voyants lumineux, par exemple un écran à cristaux liquides.
- les moyens de détermination de durée de consommation de gaz comprennent un contrôleur électronique.
- les moyens d'interruption de flux comprennent une valve ou vanne, de préférence commandée par les moyens de traitement d'information, en particulier une électrovanne.
- il comporte en outre une source d'énergie alimentant au moins les moyens d'affichage et les moyens de traitement, de préférence la source d'énergie comporte une ou plusieurs piles ou batteries électriques.
- l'organe rotatif est une hélice ou une roue.
- l'organe rotatif est apte à et conçu pour être mis en rotation par un flux gazeux, typiquement de l'air ou de l'oxygène, compris entre 0,1 et 20 l/min, de préférence entre 0,5 et 15 l/min.
- l'organe rotatif comprenant au moins un aimant qui permet au capteur Hall de comptabiliser le nombre de tours pendant une durée donnée.

L'invention concerne aussi un procédé de mesure de la durée de consommation gazeuse au sein d'un bâtiment hospitalier comprenant un réseau de canalisations de gaz comprenant des prises murales de distribution de gaz, dans lequel on mesure la durée de consommation gazeuse d'au moins une desdites prises murales de distribution de gaz au moyen d'un dispositif selon l'invention.

De préférence, le réseau de canalisations de gaz comprend :
- une ou des canalisations d'oxygène véhiculant de l'oxygène gazeux entre une source d'oxygène et une ou plusieurs prises murales de distribution d'oxygène, et/ou
- une ou des canalisations d'air véhiculant de l'air entre une source d'air et une ou plusieurs prises murales de distribution d'air.

Avantageusement, les prises murales de distribution de gaz sont agencées dans une ou plusieurs chambres du bâtiment hospitalier.

En outre, la source d'air et/ou la source d'oxygène sont des réservoirs de stockage d'air ou d'oxygène, respectivement, ou une unité PSA ou VSA de production d'air ou d'oxygène à partir d'air atmosphérique.

La présente invention va maintenant être décrite plus en détail en références aux Figures annexées parmi lesquelles :
- la Figure 1 schématise le principe de fonctionnement des moyens de détermination de durée de consommation de gaz équipant le dispositif de la Figure 3,
- la Figure 2 schématise l'intérieur du dispositif de la Figure 1 et
- la Figure 3 schématise le principe de fonctionnement d'un mode de réalisation d'un dispositif de mesure de durée de consommation gazeuse selon l'invention.

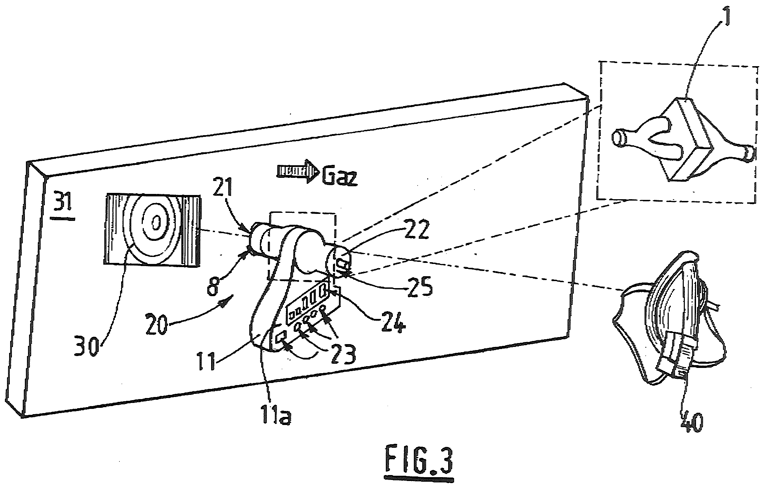

La Figure 3 schématise le principe de fonctionnement d'un mode de réalisation d'un dispositif 20 de mesure de durée de consommation d'un gaz par un patient selon l'invention, en particulier un gaz comme l'oxygène médical ou l'air médical qui sont des gaz habituellement amenés jusque dans les salles de soins ou les chambres des patients en hôpital, via des réseaux de canalisations de gaz et qui y sont distribués par des prises murales 30 portées par les murs ou parois 31, avant d'être administrés aux patients par l'intermédiaire d'interfaces, tels les masques respiratoires 40.

Ce dispositif 20 de détermination de la durée de consommation comprend un boitier 11 traversé par un passage interne de gaz 25 avec un orifice d'entrée 21 de gaz et un orifice de sortie 22 de gaz, le gaz circulant dans le sens de la flèche « GAZ », c'est-à-dire dans le sens allant de l'entrée 21 vers la sortie 22 de gaz.

Au niveau de l'orifice d'entrée 21 de gaz, le boitier 11 comprend des moyens de connexion 8, par exemple un filetage, un système à détrompage du type male/femelle ou analogue, permettant de connecter fluidiquement le dispositif à la prise de distribution de gaz 30.

Sont également prévus des moyens de réglage qui servent à régler une durée de consommation de gaz souhaitée T_{fixé}, c'est-à-dire à indiquer au dispositif le temps pendant lequel il doit laisser circuler du gaz dans le passage interne 25 de manière à alimenter le patient en gaz respiratoire selon la prescription faite par le médecin ou analogue.

Les moyens de réglage 23 comprennent un ou plusieurs organes de réglage, tel que touches, boutons, clavier numérique, curseurs ou autre.

Le réglage de la durée de consommation de gaz souhaitée T_{fixé} est préférentiellement opéré par le personnel soignant, par exemple un médecin ou une infirmière.

Des moyens d'affichage 24, tel un écran, permettent d'afficher la durée de consommation de gaz souhaitée T_{fixé} ou toute autre information ou donnée désirée. De préférence, l'écran 24 est situé sur la face avant 11a du boitier 11, c'est-à-dire la face 11a située du côté de l'orifice de sortie 22.

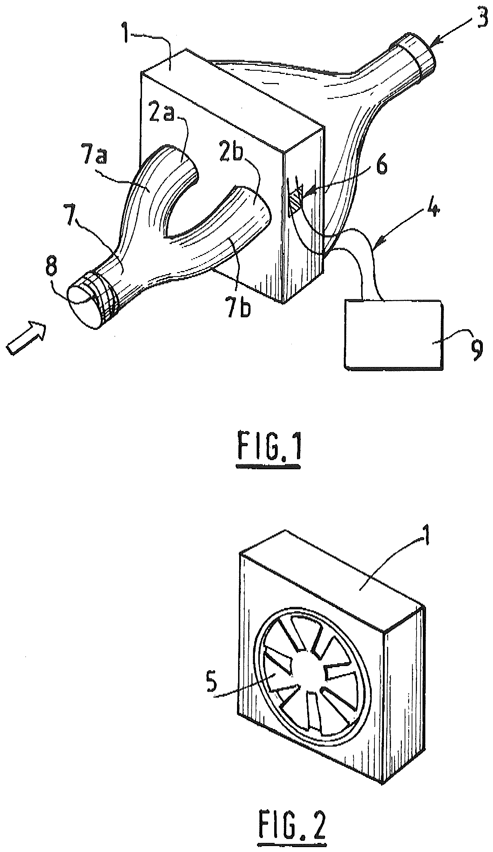

Par ailleurs, le boitier 11 comporte aussi des moyens de détermination de durée de consommation de gaz 1 conçus pour mesurer la durée totale T_{conso} de consommation de gaz, c'est-à-dire le temps total pendant lequel du gaz circule dans le passage de gaz de l'entrée 21 de gaz vers la sortie 22 de gaz, avant d'être envoyé via un tuyau flexible ou analogue vers l'interface 40 reliée au patient. Un mode de réalisation de moyens de détermination de durée de consommation de gaz 1 est détaillé ci-après et illustré sur les Figures 1 et 2.

Dans le boitier 1, on prévoit en outre des moyens de traitement d'information aptes à et conçus pour comparer la durée (T_{conso}) totale de consommation de gaz déterminée par les moyens de détermination de durée de consommation de gaz 1 à la durée de consommation de gaz souhaitée T_{fixé}. Les moyens de traitement d'information sont par exemple une carte électronique organisée autour d'un microcontrôleur programmable, ou une transmission à distance ee type wifi/zigbee de la consommation vers une console centrale.

Enfin, le dispositif de l'invention 20 comprend également des moyens d'interruption de flux, telle une électrovanne, qui sont conçus pour interrompre toute circulation de gaz dans le passage interne de gaz 25 lorsque les moyens de traitement d'information déterminent que la durée (T_{conso}) totale de consommation de gaz est supérieure ou égale à la durée de consommation de gaz souhaitée (T_{fixé}).

La Figure 1 schématise le principe de fonctionnement des moyens de détermination de durée de consommation de gaz équipant le dispositif de la Figure 3.

Lesdits moyens de détermination de durée de consommation de gaz comprennent un compartiment ou chambre 1 avec une ou plusieurs entrées de gaz 2 et une sortie de gaz 3, et dans lequel compartiment ou chambre 1 est agencé un organe rotatif 5, telle une hélice, comme expliqué ci-après.

Ici, le compartiment 1 comprend deux entrées 2a, 2b de gaz alimentées chacune par un conduit d'alimentation unique 7. Ce conduit d'alimentation unique 7 se ramifie en deux sous-branche 7a, 7b qui alimentent chacune l'une desdites entrées 2a, 2b du compartiment 1. L'intérêt d'avoir plusieurs entrées 2a, 2b est de pouvoir mettre en mouvement l'hélice 5 même lorsque les débits de gaz sont faibles, c'est-à-dire typiquement à partir de 0,5 l/min environ, grâce à une répartition du flux gazeux sur plusieurs pales de l'hélice 5.

Le gaz traverse le compartiment ou chambre 1 comprenant l'hélice 5 et en ressort ensuite par un conduit de sortie 17 portant la sortie de gaz 3. Le conduit d'alimentation unique 7 et le conduit de sortie 17 viennent chacun se raccorder et/ou forment une portion du passage interne de gaz 25 traversant le boitier 11 du dispositif 20 de l'invention.

Typiquement, les moyens de mesure de vitesse de flux gazeux comprennent un organe rotatif 5, telle une hélice ou une roue rotative, apte à et conçu pour être mis en rotation par le flux gazeux lorsque celui-ci traverse le compartiment 1 dans le sens (voir Flèche en Fig. 1) allant des entrées 2 vers la sortie 3.

L'organe rotatif 5 coopère avec un capteur à effet hall 6 de manière à déterminer la durée et/ou la vitesse de rotation de l'organe rotatif 5 grâce au comptage du nombre de tours que fait l'organe rotatif 5 pendant un temps donné, c'est-à-dire pendant le temps de mesure.

La durée de rotation de l'organe rotatif 5 correspond en fait à la durée pendant laquelle du gaz traverse le dispositif 20, donc la durée pendant laquelle le gaz thérapeutique est administré au patient, c'est-à-dire la durée T_{conso} totale de consommation de gaz.

Les mesures opérées par l'organe rotatif 5 sont ensuite transmises, via des connexions électriques 4, à des moyens de traitement d'information 9, tel contrôleur électronique, compris dans le boitier 11 du dispositif 20 de l'invention de sorte de déterminer la durée T_{conso} totale de consommation de gaz à partir des mesures de vitesse de rotation (i. e. nombre de tours pendant un temps donné) de l'organe rotatif 5, et de comparer cette durée T_{conso} totale de consommation de gaz avec la durée de consommation de gaz souhaitée T_{fixé}, de manière à interrompre toute circulation de gaz dans le passage interne 25 de gaz lorsque T_{conso} ≥ T_{fixé}.

L'interruption de toute circulation de gaz dans le passage interne 25 de gaz est obtenue en agissant sur l'électrovanne agencée sur le passage interne 25, ladite électrovanne étant commandée par les moyens de traitement d'information, notamment le contrôleur électronique 9.

Le contrôleur électronique 9, l'afficheur 24 et l'électrovanne agencée sur le passage interne 25 de gaz sont alimentés en courant électrique par une pile, une batterie ou le secteur.

Les données peuvent être ensuite transmises par le contrôleur électronique 9 ou un autre moyen de transmission, par voie filaire ou sans fil, par exemple une transmission wifi, zigbee, bluetooth ou autre, vers un système d'information stockant et/ou traitant les données recueillies, par exemple un ordinateur, un serveur ou autre.

De façon alternative, des moyens d'enregistrement, par exemple une carte mémoire, permettent de stocker les données mesurées ou calculées à partir des mesures.

Le dispositif de l'invention est particulièrement bien adapté au suivi de la consommation gazeuse des prises murales agencées dans les chambres d'hôpital ou analogue, en particulier d'air médical ou d'oxygène médical, au sein d'un bâtiment hospitalier, typiquement en tête de lit.

## Revendications

1. Dispositif (20) de détermination de la durée de consommation d'un gaz par un patient comprenant :
- un passage interne de gaz (25) avec une entrée de gaz (21) et une sortie de gaz (22),
- des moyens de connexion (8) permettant de connecter fluidiquement le dispositif à une prise de distribution de gaz (30),
- des moyens de réglage (23) permettant de régler une durée de consommation de gaz souhaitée(T_{fixé}),
- des moyens d'affichage (24) permettant d'afficher au moins la durée de consommation de gaz souhaitée (T_{fixé}),
- des moyens de détermination de durée de consommation de gaz (5, 6) conçus pour mesurer la durée totale (T_{conso}) de consommation de gaz pendant laquelle du gaz circule dans ledit passage interne de gaz (25), de l'entrée de gaz (21) vers la sortie de gaz (22),
- des moyens de traitement d'information (9) aptes à et conçus pour comparer la durée (T_{conso}) totale de consommation de gaz déterminée par les moyens de détermination de durée de consommation de gaz (5, 6) à la durée de consommation de gaz souhaitée (T_{fixé}), et
- des moyens d'interruption de flux conçus pour interrompre toute circulation de gaz dans ledit passage de gaz lorsque les moyens de traitement d'information déterminent que la durée (T_{conso}) totale de consommation de gaz est supérieure ou égale à la durée de consommation de gaz souhaitée (T_{fixé}).

2. Dispositif selon la revendication 1, **caractérisé en ce que** les moyens de détermination de durée de consommation de gaz (5, 6) comprennent :
- un organe rotatif (5) apte à et conçu pour être mis en rotation par le flux gazeux circulant dans le passage interne de gaz (25), et
- un capteur à effet hall (6) coopérant avec l'organe rotatif (5) pour comptabiliser la durée pendant laquelle l'organe rotatif (5) est mis en rotation par le flux gazeux circulant dans ledit passage interne de gaz (25).

3. Dispositif selon l'une des revendications précédentes, **caractérisé en ce que** les moyens de réglage (23) comprennent un ou plusieurs organes de réglage.

4. Dispositif selon l'une des revendications précédentes, **caractérisé en ce que** les moyens d'affichage (24) comprennent un écran ou des voyants lumineux.

5. Dispositif selon l'une des revendications précédentes, **caractérisé en ce que** les moyens de détermination de durée de consommation de gaz comprennent un contrôleur électronique (9).

6. Dispositif selon l'une des revendications précédentes, **caractérisé en ce que** les moyens d'interruption de flux comprennent une valve.

7. Dispositif selon l'une des revendications précédentes, **caractérisé en ce que** les moyens d'interruption de flux comprennent une électrovalve.

8. Dispositif selon l'une des revendications précédentes, **caractérisé en ce qu'**il comporte en outre une source d'énergie alimentant au moins les moyens d'affichage et les moyens de traitement, de préférence la source d'énergie comporte une ou plusieurs piles ou batteries électriques.

9. Dispositif selon l'une des revendications précédentes, **caractérisé en ce que** l'organe rotatif (5) est une hélice ou une roue, de préférence l'organe rotatif (5) comprenant au moins un aimant.

10. Procédé de mesure de la durée de consommation gazeuse au sein d'un bâtiment hospitalier comprenant un réseau de canalisations de gaz comprenant des prises murales de distribution de gaz, dans lequel on mesure la durée de consommation gazeuse d'au moins une desdites prises murales de distribution de gaz au moyen d'un dispositif selon l'une des revendications précédentes.

11. Procédé selon la revendication 10, **caractérisé en ce que** le réseau de canalisations de gaz comprend :
- une ou des canalisations d'oxygène véhiculant de l'oxygène gazeux entre une source d'oxygène et une ou plusieurs prises murales de distribution d'oxygène, et/ou
- une ou des canalisations d'air véhiculant de l'air entre une source d'air et une ou plusieurs prises murales de distribution d'air.

12. Procédé selon l'une des revendications 10 ou 11, **caractérisé en ce que** les prises murales de distribution de gaz sont agencées dans une ou plusieurs chambres du bâtiment hospitalier.

## Patentansprüche

1. Vorrichtung (20) zur Bestimmung der Verbrauchsdauer eines Gases durch einen Patienten, umfassend:
- einen internen Gasdurchgang (25) mit einem Gaseinlass (21) und einem Gasauslass (22),
- Anschlussmittel (8), die es ermöglichen, die Vorrichtung fluidisch mit einer Steckdose zur Gasverteilung (30) zu verbinden,
- Einstellmittel (23), die es ermöglichen, eine gewünschte Gasverbrauchsdauer (T_{fixiert}) einzustellen,
- Anzeigemittel (24), die es ermöglichen, mindestens die gewünschte Gasverbrauchsdauer (T_{fixiert}) anzuzeigen,
- Mittel zur Bestimmung einer Gasverbrauchsdauer (5, 6), die ausgeführt sind, um die Gesamtdauer (T_{Verbr.}) eines Gasverbrauchs zu messen, während der Gas in dem internen Gasdurchgang (25) vom Gaseinlass (21) zum Gasauslass (22) strömt,
- Informationsbearbeitungsmittel (9), die imstande und ausgeführt sind, um die Gesamtdauer (T_{Verbr.}) eines Gasverbrauchs, die durch die Mittel zur Bestimmung einer Gasverbrauchsdauer (5, 6) bestimmt wird, mit der gewünschten Gasverbrauchsdauer (T_{fixiert}) zu vergleichen, und
- Strömungsunterbrechungsmittel, die ausgeführt sind, um die gesamte Gaszirkulation in dem Gasdurchgang zu unterbrechen, wenn die Informationsbearbeitungsmittel bestimmen, dass die Gesamtdauer (T_{Verbr.}) eines Gasverbrauchs größer oder gleich der gewünschten Gasverbrauchsdauer (T_{fixiert}) ist.

2. Vorrichtung nach Anspruch 1, **dadurch gekennzeichnet, dass** die Mittel zur Bestimmung einer Gasverbrauchsdauer (5, 6) umfassen:
- ein Drehorgan (5), das imstande und ausgeführt ist, um durch die Gasströmung, die in dem internen Gasdurchgang (25) zirkuliert, in Drehung versetzt zu werden, und
- einen Hall-Sensor (6), der mit dem Drehorgan (5) zusammenwirkt, um die Dauer aufzuzeichnen, während der das Drehorgan (5) durch die Gasströmung, die in dem internen Gasdurchgang (25) zirkuliert, in Drehung versetzt wird.

3. Vorrichtung nach einem der vorstehenden Ansprüche, **dadurch gekennzeichnet, dass** die Einstellmittel (23) ein oder mehrere Einstellorgane umfassen.

4. Vorrichtung nach einem der vorstehenden Ansprüche, **dadurch gekennzeichnet, dass** die Anzeigemittel (24) einen Bildschirm oder Kontrollleuchten umfassen.

5. Vorrichtung nach einem der vorstehenden Ansprüche, **dadurch gekennzeichnet, dass** die Mittel zur Bestimmung einer Gasverbrauchsdauer einen elektronischen Regler (9) umfassen.

6. Vorrichtung nach einem der vorstehenden Ansprüche, **dadurch gekennzeichnet, dass** die Strömungsunterbrechungsmittel ein Ventil umfassen.

7. Vorrichtung nach einem der vorstehenden Ansprüche, **dadurch gekennzeichnet, dass** die Strömungsunterbrechungsmittel ein Magnetventil umfassen.

8. Vorrichtung nach einem der vorstehenden Ansprüche, **dadurch gekennzeichnet, dass** sie weiter eine Energiequelle umfasst, die mindestens die Anzeigemittel und die Bearbeitungsmittel versorgt, wobei die Energiequelle vorzugsweise eine oder mehrere elektrische Zellen oder Batterien umfasst.

9. Vorrichtung nach einem der vorstehenden Ansprüche, **dadurch gekennzeichnet, dass** das Drehorgan (5) eine Spirale oder ein Rad ist, wobei das Drehorgan (5) vorzugsweise mindestens einen Magneten umfasst.

10. Verfahren zur Messung der Dauer eines Gasverbrauchs innerhalb eines Spitalgebäudes, ein Gasleitungsnetz umfassend, das Wandsteckdosen zur Gasverteilung umfasst, wobei man die Gasverbrauchsdauer zumindest einer der Wandsteckdosen zur Gasverteilung anhand einer Vorrichtung nach einem der vorstehenden Ansprüche misst.

11. Verfahren nach Anspruch 10, **dadurch gekennzeichnet, dass** das Gasleitungsnetz umfasst:
- eine oder mehrere Sauerstoffleitungen, die gasförmigen Sauerstoff zwischen einer Sauerstoffquelle und einer oder mehreren Wandsteckdosen zur Sauerstoffverteilung befördern, und/ oder
- eine oder mehrere Luftleitungen, die Luft zwischen einer Luftquelle und einer oder mehreren Wandsteckdosen zur Luftverteilung befördern.

12. Verfahren nach einem der Ansprüche 10 oder 11, **dadurch gekennzeichnet, dass** die Wandsteckdosen zur Gasverteilung in einem oder mehreren Zimmern des Spitalgebäudes angeordnet sind.

## Claims

1. Device (20) for determining the consumption period of a gas by a patient comprising:
- an internal gas passage (25) with a gas inlet (21) and a gas outlet (22),
- connection means (8) enabling the smooth connection of the device to a gas dispersion socket (30),
- adjustment means (23) allowing a desired gas consumption period (T_{fixed}) to be adjusted,
- display means (24) allowing at least the gas consumption period (T_{fixed}) to be displayed,
- means for determining the gas consumption period (5, 6) designed to measure the total gas consumption period (T_{consumption}) during which the gas circulates in said internal gas passage (25), from the gas inlet (21) towards the gas outlet (22),
- means for processing information (9) able to and designed to compare the total gas consumption period (T_{consumption}) determined by the means for determining the gas consumption period (5, 6) with the desired gas consumption period (T_{fixed}), and
- means for interrupting flow, designed to interrupt any gas circulation in said gas passage when the means for processing information determine that the total gas consumption period (T_{consumption}) is longer than or equal to the desired gas consumption period (T_{fixed}).

2. Device according to claim 1, **characterised in that** the means for determining the gas consumption period (5, 6) comprise:
- a rotating body (5) able to and designed to be rotated by the gaseous flow circulating in the internal gas passage (25), and
- a Hall effect sensor (6) cooperating with the rotating body (5) to account for the period during which the rotating body (5) is rotated by the gaseous flow circulating in said internal gas passage (25).

3. Device according to one of the preceding claims, **characterised in that** the adjustment means (23) comprise one or a plurality adjustment bodies.

4. Device according to one of the preceding claims, **characterised in that** the display means (24) comprise a screen or indicator lights.

5. Device according to one of the preceding claims, **characterised in that** the means for determining the gas consumption period comprise an electronic controller (9).

6. Device according to one of the preceding claims, **characterised in that** the flow interruption means comprise a valve.

7. Device according to one of the preceding claims, **characterised in that** the flow interruption means comprise an electrovalve.

8. Device according to one of the preceding claims, **characterised in that** it further comprises an energy source supplying at least the display means and processing means, preferably the energy source comprises one or a plurality batteries or electric batteries.

9. Device according to one of the preceding claims, **characterised in that** the rotating body (5) is a helix or a wheel, preferably the rotating body (5) comprising at least one magnet.

10. Method for measuring the gas consumption period within a hospital building comprising a network of gas channels comprising gas dispersion wall sockets, wherein the gas consumption period is measured by at least one of said gas dispersion wall sockets by means of a device according to one of the preceding claims.

11. Method according to claim 10, **characterised in that** the gas channel network comprises:
- one or a plurality oxygen channels transmitting gaseous oxygen between an oxygen source and one or a plurality oxygen dispersion wall sockets, and/or
- one or a plurality air channels transmitting air between an air source and one or a plurality air dispersion wall sockets.

12. Method according to one of claims 10 or 11, **characterised in that** the gas dispersion wall sockets are arranged in one or a plurality of hospital building rooms.
